# FASCICULE DE BREVET EUROPEEN

(11) **EP 3 340 909 B1**
(45) Date de publication et mention de la délivrance du brevet: **31.07.2019**
(21) Numéro de dépôt: 16763894.9
(22) Date de dépôt: 08.08.2016
(51) Int. Cl.: A61B 17/70, A61B 17/88

(54) **ENSEMBLE CHIRURGICAL POUR LA MISE EN PLACE D'UN BOUCHON DE VIS PEDICULAIRE**
CHIRURGISCHE ANORDNUNG ZUM PLATZIEREN EINER PEDIKELSCHRAUBENABDECKUNG
SURGICAL ASSEMBLY FOR PLACING A PEDICLE SCREW COVER

(30) Priorité: 27.08.2015 FR 1557966
(43) Date de publication de la demande: 04.07.2018
(73) Titulaire: Spineway, 69130 Ecully (FR)
(72) Inventeur: BAZILLE, Julien, 69009 Lyon (FR)
(74) Mandataire: Cabinet Laurent & Charras
(86) Numéro de dépôt international: PCT/FR2016/052049
(87) Numéro de publication internationale: WO 2017/032938

(56) Documents cités:
- WO-A1-00/22997
- WO-A1-94/26190
- WO-A1-2008/134758
- FR-A1- 2 863 861
- US-A1- 2014 214 097

## Description

### DOMAINE TECHNIQUE

La présente invention concerne le secteur technique des instruments chirurgicaux et concerne plus particulièrement un ensemble chirurgical pour la mise en place d'un bouchon dans une vis pédiculaire, notamment pour le traitement des pathologies du rachis.

### ETAT ANTERIEUR DE LA TECHNIQUE

D'une manière générale, un implant rachidien comprend au moins deux vis pédiculaires destinées à être vissées sur un élément anatomique variable de la vertèbre (lame, pédicule, corps vertébral), et un système de liaison (plaque ou tige) unissant les deux vis pédiculaires entre elles.

Il est parfaitement connu d'utiliser un bouchon fileté adapté pour venir se visser dans la tête de la vis pédiculaire et pour maintenir en place une tige de liaison. Le bouchon utilisé se présente soit sous la forme d'un bouchon simple comprenant un filetage externe, et un évidement central de serrage comprenant, par exemple, une connexion pour un embout de vissage étoilé, soit sous la forme d'un bouchon double comprenant un bouchon interne et un bouchon externe vissés l'un dans l'autre. Le bouchon interne comprend un évidement central de serrage et un filetage externe, et le bouchon externe comprend un filetage interne et un filetage externe et des évidements de serrage.

Il est également connu des instruments pour la mise en place de ces bouchons. Ces instruments forment, par exemple, un ensemble comprenant généralement le bouchon en tant que tel, et un porte-bouchon. Les instruments sont notamment conçus pour des opérations chirurgicales dites mini-invasives, lors desquelles le chirurgien ne dispose d'aucune visibilité sur les implants.

Il est connu de l'état de la technique un ensemble chirurgical comprenant un bouchon et un porte-bouchon, notamment divulgué dans le document US 2011166610. Le porte-bouchon comprend deux branches longitudinales élastiques et diamétralement opposées, entourées par un manchon coulissant. Les branches comprennent chacune un bossage interne en regard l'un de l'autre. Le bouchon comprend deux fentes radiales diamétralement opposées, et ménagées à partir d'une paroi latérale externe dudit bouchon. Les deux fentes comprennent chacune un évidement complémentaire au bossage des branches, ménagé en direction de l'intérieur du bouchon.

Afin de porter le bouchon, les deux branches viennent, par déformation élastique, dans les fentes avec leur bossage en engagement dans les évidements complémentaires pour enserrer le bouchon. Le manchon du porte-bouchon est coulissé en direction du bouchon pour empêcher les branches élastiques de s'écarter l'une de l'autre et verrouiller la connexion entre le porte-bouchon et le bouchon.

Il est également connu un bouchon et un porte-bouchon, notamment divulgués dans le document US 2012123431. Le bouchon comprend un évidement central avec une rainure annulaire interne. Le porte-bouchon comprend un manchon creux présentant une pluralité de fentes longitudinales définissant des branches élastiques. Les branches élastiques comprennent chacune un bourrelet faisant saillie vers l'extérieur du manchon, apte à venir se loger, par la déformation élastique des branches, à l'intérieur de la rainure annulaire du bouchon. L'insertion d'une tige de blocage dans le manchon creux permet de verrouiller la connexion entre le porte-bouchon et le bouchon en empêchant le rapprochement des branches élastiques.

Sont également connus du document US 2014214097 un bouchon évidé et son porte-bouchon creux comprenant une extrémité de connexion, une tige de blocage montée coulissante et présentant des encoches et des ergots permettant de bloquer le bouchon. Le document WO 2008/134758 décrit un ensemble chirurgical d'un type analogue permettant de bloquer radialement le manchon sur le bouchon par le moyen d'un mécanisme comprenant des encoches radiales, deux rainures latérales opposées ainsi que deux ergots avec des épaulements latéraux complémentaires aux rainures.

Un inconvénient des ensembles chirurgicaux de l'art antérieur décrits ci-avant réside dans le fait qu'ils ne peuvent pas s'adapter aux deux types de bouchon, simple ou double, en permettant à la fois le vissage du bouchon interne et le vissage du bouchon externe. En effet, l'état de la technique décrit ci-avant ne permet pas au chirurgien d'exercer, par l'intermédiaire du porte-bouchon, un couple de serrage conséquent sur le bouchon, quel que soit son type.

### EXPOSE DE L'INVENTION

L'un des buts de l'invention est donc de remédier au moins aux inconvénients précités en proposant un ensemble chirurgical pour la mise en place d'un bouchon simple ou double dans une vis pédiculaire, dont la connexion entre le bouchon et le porte-bouchon est relativement sécurisée pour éviter les risques de déconnexion durant la mise en place du bouchon, autorisant une reconnexion facilitée in-situ du bouchon et du porte-bouchon, tout en étant suffisamment résistante pour permettre au chirurgien d'exercer, par l'intermédiaire du porte-bouchon, un couple de serrage important sur le bouchon, à savoir au minimum équivalent à 5 Nm.

Un autre objectif de la présente invention est de renforcer le verrouillage entre le bouchon et le porte-bouchon et de sécuriser davantage leur connexion.

A cet effet, il a été mis au point un ensemble chirurgical comprenant :
- un bouchon de vis pédiculaire, le bouchon comprenant au moins un filetage externe et un évidement central pour l'engagement d'un embout de vissage ;
- un porte-bouchon sous forme d'un manchon creux comprenant une extrémité de connexion avec le bouchon, et une tige de blocage montée coulissante dans le manchon creux entre une position neutre et une position de blocage du bouchon.

Selon l'invention :
- le bouchon comprend au moins deux encoches radiales diamétralement opposées, ménagées à partir d'une paroi latérale externe dudit bouchon, chacune des encoches comprenant au moins une rainure latérale ;
- l'extrémité de connexion du manchon du porte-bouchon se prolonge longitudinalement par deux ergots diamétralement opposés aptes à venir s'engager dans les encoches et comprenant chacun un épaulement latéral complémentaire à la rainure de l'encoche apte à s'engager dans ladite rainure pour bloquer axialement le manchon sur le bouchon.

Par la direction latérale des rainures et des épaulements, on entend une direction perpendiculaire à la direction radiale respectivement du bouchon ou du manchon. De cette manière, la présente invention permet de saisir, par l'intermédiaire du porte-bouchon, un bouchon pour sa mise en place dans une vis pédiculaire. En pratique, dans cette forme de réalisation, la saisie du bouchon s'effectue en insérant les ergots du manchon dans les encoches dudit bouchon, et en tournant ledit manchon dans le sens du vissage pour engager les épaulements dans les rainures et bloquer axialement le manchon sur le bouchon. Pour maintenir ce blocage axial de manière sécurisée, il convient de coulisser la tige de blocage dans la position de blocage, c'est-à-dire jusqu'à ce que ladite tige de blocage vienne exercer une pression axiale sur le bouchon pour maintenir les épaulements en engagement dans les rainures.

L'invention présente l'avantage de pouvoir être mise en oeuvre avec un bouchon simple comprenant un filetage externe et un évidement central de serrage avec les encoches ménagées autour de l'évidement central, ou avec un bouchon double comprenant les encoches ménagées dans le bouchon externe, autour du bouchon interne.

De cette manière, les ergots épaulés permettent au chirurgien d'exercer, par l'intermédiaire du porte-bouchon, un couple de serrage important sur le bouchon simple ou sur le bouchon externe du double bouchon, à savoir au minimum un couple de serrage équivalent à 5 Nm.

Pour visser le bouchon interne, il convient de déconnecter et de retirer le porte-bouchon puis d'insérer un organe de vissage dans l'évidement de serrage pour le vissage en tant que tel.

La connexion/déconnexion entre le bouchon et le porte-bouchon est simple. De plus, la configuration du porte-bouchon est telle qu'il présente, en pratique, un manchon comprenant un diamètre inférieur à 10 mm pour pouvoir s'insérer dans un tube d'extension utilisé pour la mise en place d'une vis pédiculaire.

Avantageusement, les encoches et les ergots comprennent chacune et chacun respectivement une deuxième rainure latérale opposée et un deuxième épaulement latéral opposé, ce qui permet un blocage axial du porte-bouchon sur le bouchon également dans le sens du dévissage du bouchon.

Dans cette dernière configuration, et selon l'invention, l'extrémité de connexion du manchon comprend deux fentes longitudinales diamétralement opposées de sorte à former deux branches élastiques, lesdites fentes passant par les ergots pour former deux paires d'ergots. Les ergots de chaque paire d'ergots sont aptes à venir s'engager, en se rapprochant l'un de l'autre par la déformation élastique des branches, dans l'encoche correspondante jusqu'à ce que les épaulements des ergots viennent s'encliqueter dans les rainures des encoches.

Cette configuration permet de verrouiller de manière plus sécurisée et par encliquetage la connexion entre le porte-bouchon et le bouchon.

Selon une forme de réalisation particulière, les rainures des encoches et les épaulements des ergots sont inclinés de sorte que la rotation du manchon dans le sens du vissage force le blocage axial.

Selon une forme de réalisation particulière, le bouchon comprend deux encoches radiales supplémentaires, diamétralement opposées et régulièrement réparties de part et d'autre des premières encoches, et le manchon comprend des pattes de serrage prolongeant longitudinalement l'extrémité de connexion du manchon, diamétralement opposées et régulièrement réparties de part et d'autre des ergots, lesdites pattes de serrage étant destinées à venir s'engager de manière ajustée dans les encoches pour permettre de faciliter le vissage/dévissage du bouchon en transmettant de manière optimale audit bouchon le couple de serrage/desserrage exercé par le manchon creux.

Dans cette forme de réalisation, et afin de sécuriser davantage la connexion entre le bouchon et le porte-bouchon, la tige de blocage comprend un diamètre externe ajusté au diamètre interne du manchon creux, de sorte qu'en position de blocage la tige de blocage est positionnée entre les branches élastiques du manchon et empêche le déverrouillage du porte-bouchon en interdisant le rapprochement des branches élastiques et le désengagement des paires d'ergots.

D'une manière avantageuse, la tige de blocage est assujettie en partie supérieure à une gâchette pour le passage de la tige de blocage de la position neutre jusqu'à la position de blocage.

Selon une forme de réalisation particulière, la tige de blocage comprend un embout de vissage apte à venir en engagement, en position de blocage, dans l'évidement central pour le vissage du bouchon.

Ainsi, le bouchon interne peut être vissé avec la tige de blocage en positon de blocage. Il n'est donc pas nécessaire de retirer le porte-bouchon pour visser le bouchon interne, ni d'utiliser un organe de serrage distinct.

Selon une autre forme de réalisation avantageuse, la tige de blocage est creuse et permet l'insertion d'un organe de vissage sous la forme d'une tige avec un embout de vissage. Ainsi, le vissage du bouchon interne peut être effectué sans retirer le porte-bouchon.

De préférence, les pattes de serrage sont plus longues que les ergots afin de permettre le guidage de l'engagement desdits ergots dans les encoches.

Comme évoqué ci-avant, le bouchon se présente soit sous la forme d'un bouchon simple, soit sous la forme d'un bouchon double. Dans le cas d'un bouchon double, le bouchon interne comprend un bourrelet annulaire inférieur formant une butée lors du dévissage du bouchon interne, ou lors du vissage du bouchon externe empêchant ainsi le désassemblage accidentel des deux bouchons pendant l'opération chirurgicale.

### BREVE DESCRIPTION DES DESSINS

D'autres caractéristiques et avantages de l'invention ressortiront clairement de la description qui en est réalisée ci-après, à titre indicatif et nullement limitatif, en référence aux figures annexées dans lesquelles :
- la figure 1 est une représentation schématique en perspective d'un bouchon simple selon l'invention;
- la figure 2 est une représentation schématique illustrant le bouchon simple vue de côté ;
- la figure 3 est une représentation schématique en perspective d'un bouchon double selon l'invention ;
- la figure 4 est une représentation schématique en perspective d'un bouchon double vue de côté ;
- la figure 5 est une représentation schématique illustrant le bouchon double vue en coupe longitudinale ;
- la figure 6 est une vue schématique en perspective de l'extrémité de connexion du porte-bouchon selon l'invention ;
- la figure 7 est une représentation schématique vue de côté illustrant les paires d'ergots du porte-bouchon avant engagement dans les encoches d'un bouchon double ;
- la figure 8 est une représentation schématique vue de côté illustrant les pattes de serrage du porte-bouchon avant engagement dans les encoches d'un bouchon double ;
- la figure 9 est une représentation schématique illustrant de côté le porte-bouchon connecté au bouchon, avec une forme de réalisation particulière des ergots dudit porte-bouchon ;
- la figure 10 est une représentation schématique similaire à celle de la figure 9, illustrant une autre forme de réalisation possible des ergots du porte-bouchon ;
- la figure 11 est une représentation schématique illustrant l'extrémité de connexion du porte-bouchon et le bouchon en coupe longitudinale suivant l'axe A-A représenté sur les figures 9 et 10 ;
- la figure 12 est une vue schématique en perspective de l'ensemble chirurgical selon l'invention, la tige de blocage du porte-bouchon étant déverrouillée ;
- la figure 13 est une vue en coupe longitudinale de l'extrémité de connexion du porte-bouchon, avec la tige de blocage en position de déverrouillage ;
- la figure 14 est une vue schématique en perspective de l'ensemble chirurgical selon l'invention, la tige de blocage étant verrouillée et le porte-bouchon étant connecté au bouchon ;
- la figure 15 est une vue en coupe longitudinale de l'extrémité de connexion du porte-bouchon, avec la tige de blocage en position de verrouillage.
- la figure 16 est une vue schématique en perspective illustrant l'extrémité de connexion du porte-bouchon connectée au bouchon.

### EXPOSE DETAILLE DE L'INVENTION

L'invention concerne un ensemble chirurgical (1) pour la mise en place d'un bouchon (2) dans une vis pédiculaire, notamment pour le traitement des pathologies du rachis. L'ensemble chirurgical (1) selon l'invention comprend le bouchon (2) en tant que tel et un porte-bouchon (3).

Le bouchon (2) est adapté pour venir se visser dans la tête de la vis pédiculaire pour maintenir en place une tige de liaison d'au moins deux vis pédiculaires.

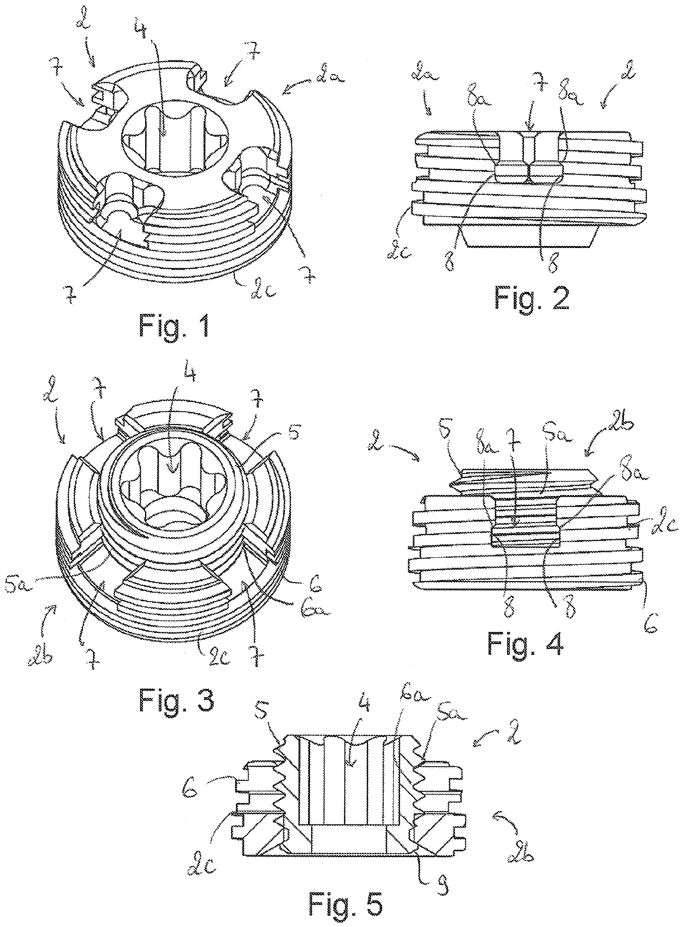

A cet effet, et en référence aux figures 1 à 2, le bouchon (2) utilisé peut se présenter sous la forme d'un bouchon simple (2a) comprenant un filetage externe (2c) apte à coopérer avec la tête de la vis pédiculaire, et un évidement central (4) de serrage comprenant par exemple une connexion pour en embout de vissage à étoile, pour le vissage en tant que tel du bouchon (2).

En référence aux figures 3 à 4, le bouchon (2) peut également se présenter sous la forme d'un bouchon double (2b) comprenant un bouchon interne (5) et un bouchon externe (6) vissés l'un dans l'autre. Le bouchon interne (5) comprend l'évidement central (4) de connexion et un filetage externe (5a) pour coopérer avec le bouchon externe (6), et le bouchon externe (6) comprend un filetage interne (6a) pour coopérer avec le bouchon interne (5), et le filetage externe (2c) pour coopérer avec la vis pédiculaire.

Selon un mode de réalisation préféré de l'invention, les deux types de bouchon (2) comprennent quatre encoches radiales identiques (7) régulièrement réparties autour de la circonférence dudit bouchon (2), et ménagées à partir d'une paroi latérale externe dudit bouchon (2). Dans le cas du bouchon simple (2a) les encoches (7) sont ménagées autour de l'évidement central (4), et dans le cas du bouchon double (2b) elles sont ménagées dans le bouchon externe (6), autour du bouchon interne (5).

Chaque encoche (7) comprend deux rainures latérales (8), c'est-à-dire ménagées dans une paroi de l'encoche (7) perpendiculaire à la direction radiale du bouchon (2). Les rainures (8) comprennent un pan supérieur (8a) incliné vers une partie inférieure du bouchon (2).

En référence à la figure 5, et dans le cas d'un bouchon double (2b), le bouchon interne (5) comprend un bourrelet annulaire inférieur (9) formant une butée lors du dévissage du bouchon interne (5), ou lors du vissage du bouchon externe (6) empêchant ainsi le désassemblage accidentel des deux bouchons (2) pendant l'opération chirurgicale.

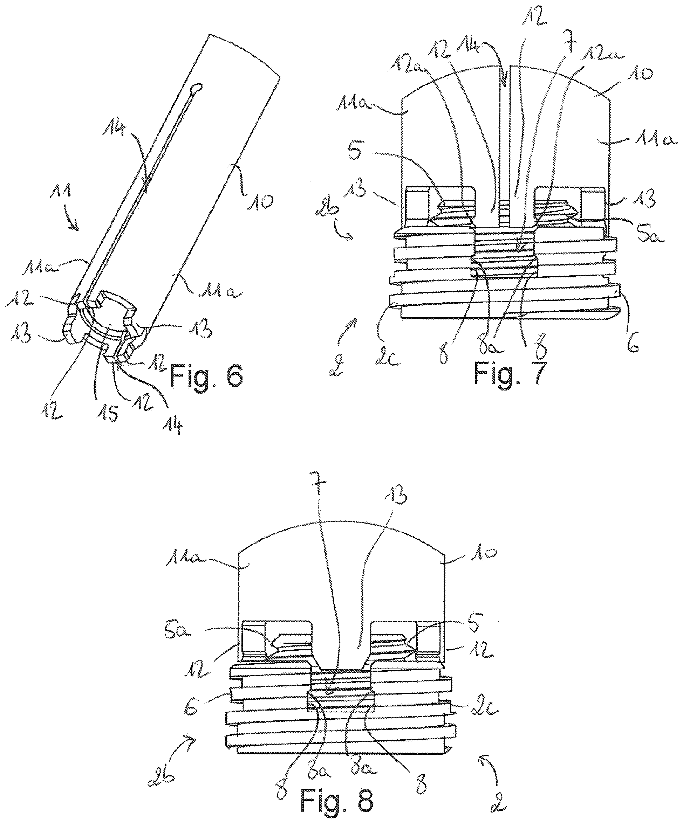

En référence aux figures 6 à 8, le porte-bouchon (3) comprend un manchon creux (10) comprenant une extrémité de connexion (11) avec le bouchon (2). L'extrémité de connexion (11) du manchon (10) se prolonge longitudinalement par deux ergots (12) diamétralement opposés et par deux pattes de serrage (13), également diamétralement opposées et régulièrement réparties de part et d'autre des ergots (12).

L'extrémité de connexion (11) comprend, en outre, deux fentes longitudinales (14) diamétralement opposées de sorte à former deux branches élastiques (11a). Les fentes (14) sont ménagées de sorte à séparer les ergots (12) en deux paires d'ergots (12). Chaque ergot (12) comprend un épaulement latéral (12a) complémentaire à la rainure (8) de l'encoche (7).

De cette manière, et en référence à la figure 7, les ergots (12) de chaque paire d'ergots (12) sont aptes à venir s'engager, en se rapprochant l'un de l'autre par la déformation élastique des branches (11a), dans l'encoche (7) correspondante jusqu'à ce que les épaulements (12a) des ergots (12) viennent s'encliqueter dans les rainures (8). La connexion entre le porte-bouchon (3) et le bouchon (2) est verrouillée de manière sécurisée et par encliquetage.

De la même manière, les deux pattes de serrage (13) sont chacune destinée à venir s'engager dans une encoche (7) correspondante. La largeur des pattes de serrage (13) correspond notamment sensiblement à la largeur des encoches (7) de sorte que l'engagement des pattes de serrage (13) est ajusté. Les pattes de serrage (13) sont plus longues que les ergots (12) afin de s'engager en premier dans les encoches (7) et permettre le guidage de l'engagement des ergots (12). Les pattes de serrage (13) permettent de transmettre de manière optimale au bouchon (2) le couple de serrage/desserrage exercé par le manchon creux (10). Cette configuration permet notamment d'exercer sur le bouchon (2) un couple de serrage conséquent, à savoir au minimum un couple de serrage équivalent à 5 Nm. Selon l'invention, le couple de serrage peut être transmis aussi bien au bouchon simple (2a) qu'au bouchon externe (6) du bouchon double (2b).

Le fait que les quatre encoches (7) soient identiques permet de connecter le porte-bouchon (3) plus facilement sur le bouchon (2), notamment in-situ. En effet, chaque ergot (12) et chaque patte de serrage (13) peut coopérer avec l'une quelconque des quatre encoches (7), ce qui autorise quatre positions possibles de connexion.

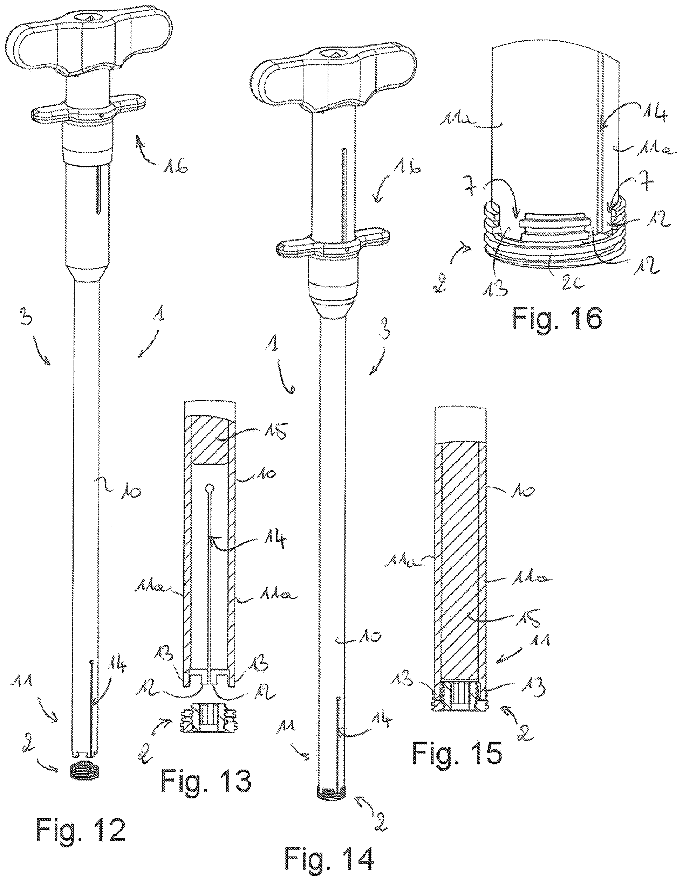

En référence aux figures 12 à 16, le porte-bouchon (3) comprend une tige de blocage (15) montée coulissante dans le manchon creux (10), entre une position neutre et une position de blocage du bouchon (2). La tige de blocage (15) comprend un diamètre externe ajusté au diamètre interne du manchon creux (10) et est assujettie en partie supérieure à une gâchette (16) pour le passage de la tige de blocage (15) de la position haute neutre (figures 12 et 13) dans laquelle elle n'empêche pas le rapprochement des branches élastiques (11a) du manchon creux (10), à une position basse de blocage (figures 14 et 15), en direction du bouchon (2), dans laquelle elle empêche le déverrouillage du porte-bouchon (3) en interdisant le rapprochement des branches élastiques (11a) et le désengagement des paires d'ergots (12). En référence à la figure 16, lorsque le porte-bouchon (3) est connecté au bouchon (2), les pattes de serrage (13) et les ergots (12) sont engagés dans les encoches (7) et le serrage du bouchon (2) est possible.

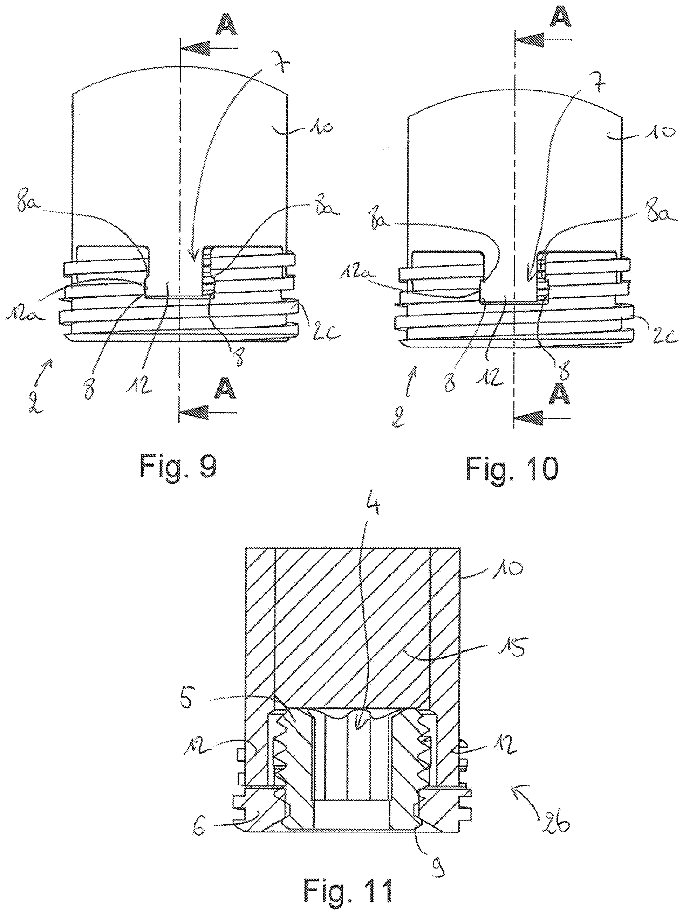

Dans une forme de réalisation particulière illustrée aux figures 9 à 11, le manchon creux (10) du porte-bouchon (3) comprend quatre ergots (12) régulièrement réparties autour de la circonférence du manchon creux (10), et ne comprend pas de pattes de serrage, ni de fentes longitudinales. La saisie du bouchon (2) s'effectue en insérant les ergots (12) dans les encoches (7) dudit bouchon (2), et en tournant ledit manchon (10) dans le sens du vissage pour bloquer axialement le manchon (10) sur le bouchon (2). Pour maintenir ce blocage axial de manière sécurisée, il convient de coulisser la tige de blocage (15) jusqu'à la position de blocage, c'est-à-dire jusqu'à ce que ladite tige de blocage (15) exerce une pression axiale sur le bouchon (2) pour maintenir les épaulements (12a) des ergots (12) en engagement dans les rainures (8) des encoches (7). Avantageusement, les épaulements (12a) des ergots (12) sont inclinés pour venir en appui contre les pans inclinés (8a) des rainures (8) de sorte que la rotation du manchon (10) dans le sens du vissage force le blocage axial.

Selon une forme de réalisation particulière illustrée à la figure 10, les pans (8a) des rainures (8) peuvent être inclinés de manière à former des systèmes de blocage en queues d'arondes, aptes à coopérer avec les épaulements complémentaires en queues d'arondes (12a) du manchon creux (10). Dans cette forme de réalisation, pour assurer le blocage du manchon (10) sur le bouchon (2), il convient de tourner le manchon (10) dans le sens du vissage ou du dévissage pour mettre en correspondance les formes en queues d'arondes, et d'exercer une pression sur le bouchon (2) avec la tige de blocage (15) pour engager lesdites formes en queues d'arondes et empêcher le déverrouillage du bouchon (2).

Selon une autre forme de réalisation non représentée, la tige de blocage (15) comprend un embout de vissage apte à venir en engagement, en position de blocage, dans l'évidement central (4) pour le vissage du bouchon (2).

En pratique, pour déconnecter le porte-bouchon (3) du bouchon (2), il convient de coulisser la tige de blocage (15) en position neutre en actionnant la gâchette (16), éventuellement d'effectuer une rotation du manchon (10) pour les formes de réalisation illustrées aux figures 9 et 10, et de tirer de part et d'autre du porte-bouchon (3) et du bouchon (2) pour les séparer l'un de l'autre. Lorsque le bouchon (2) est vissé et fixé dans la vis pédiculaire, il convient simplement, après avoir coulissé la tige de blocage (15) en position neutre, de tirer sur le porte-bouchon (3). Dans la forme de réalisation illustrée à la figure 7, les pans inclinés des épaulements (12a) des ergots (12) permettent d'exercer une force sur les branches élastiques (11a) tendant à les rapprocher l'une de l'autre. De cette manière, les paires d'ergots (12) peuvent se désengager des encoches (7). De la même manière, les épaulements (12a) sont inclinés pour permettre le rapprochement des branches élastiques (11a) lorsque les ergots (12) sont engagés dans les encoches (7).

Comme il ressort de ce qui précède, l'invention fournit un ensemble chirurgical (1) pour la mise en place d'un bouchon simple (2a) ou d'un bouchon double (2b) dans une vis pédiculaire, dont la connexion entre le bouchon (2) et le porte-bouchon (3) est renforcée et sécurisée grâce à l'encliquetage des ergots (12) et au verrouillage que procure la tige de blocage (15). Les risques de déconnexion durant la mise en place du bouchon (2) sont ainsi évités. L'ensemble chirurgical (1) selon l'invention permet une reconnexion facilitée in-situ du bouchon (2) et du porte-bouchon (3), tout en procurant une connexion suffisamment résistante pour permettre au chirurgien d'exercer, par l'intermédiaire du porte-bouchon (3), un couple de serrage conséquent sur le bouchon simple (2a) ou sur le bouchon externe (6) du double bouchon (2b), à savoir au minimum équivalent à 5 Nm.

## Revendications

1. Ensemble chirurgical (1) comprenant :
- un bouchon (2) de vis pédiculaire, le bouchon (2) comprenant au moins un filetage externe (2c) et un évidement central (4) pour l'engagement d'un embout de vissage ;
- un porte-bouchon (3) sous forme d'un manchon creux (10) comprenant une extrémité de connexion (11) avec le bouchon (2), et une tige de blocage (15) montée coulissante dans le manchon creux (10) entre une position neutre et une position de blocage du bouchon (2); et
• le bouchon (2) comprend au moins deux encoches radiales (7) diamétralement opposées, ménagées à partir d'une paroi latérale externe dudit bouchon (2), chacune des encoches (7) comprenant deux rainures latérales opposées (8) ;
• l'extrémité de connexion (11) du manchon (10) du porte-bouchon (3) se prolonge longitudinalement par deux ergots (12) diamétralement opposés aptes à venir s'engager dans les encoches (7) et comprenant chacun deux épaulements latéraux opposés (12a) complémentaires aux rainures (8) de l'encoche (7) aptes à s'engager dans les rainures (8) pour bloquer axialement le manchon (10) sur le bouchon (2) ;
**caractérisé *en ce que*** l'extrémité de connexion (11) du manchon (10) comprend deux fentes longitudinales (14) diamétralement opposées de sorte à former deux branches élastiques (11a), lesdites fentes (14) passant par les ergots (12) pour former deux paires d'ergots (12), les ergots (12) de chaque paire d'ergots (12) étant aptes à venir s'engager, en se rapprochant l'un de l'autre par la déformation élastique des branches (11a), dans l'encoche (7) correspondante jusqu'à ce que les épaulements (12a) des ergots (12) viennent s'encliqueter dans les rainures (8) des encoches (7).

2. Ensemble chirurgical (1) selon la revendication 1, ***caractérisé* en ce que** les rainures (8) des encoches (7) et les épaulements (12a) des ergots (12) sont inclinés de sorte que la rotation du manchon (10) dans le sens du vissage force le blocage axial.

3. Ensemble chirurgical (1) selon la revendication 1, ***caractérisé* en ce que** le bouchon (2) comprend deux encoches radiales (7) supplémentaires, diamétralement opposées et régulièrement réparties de part et d'autre des premières encoches (7), et le manchon (10) comprend des pattes de serrage (13) prolongeant longitudinalement l'extrémité de connexion (11) du manchon (10), diamétralement opposées et régulièrement réparties de part et d'autre des ergots (12), lesdites pattes de serrage (13) étant destinées à venir s'engager de manière ajustée dans les encoches (7).

4. Ensemble chirurgical (1) selon l'une quelconque des revendications 1 à 3, ***caractérisé* en ce que** la tige de blocage (15) comprend un diamètre externe ajusté au diamètre interne du manchon creux (10), de sorte qu'en position de blocage la tige de blocage est positionnée entre les branches élastiques (11a) du manchon (10) et empêche le déverrouillage du porte-bouchon (3) en interdisant le rapprochement des branches élastiques (11a) et le désengagement des paires d'ergots (12).

5. Ensemble chirurgical (1) selon l'une quelconque des revendications 1 à 4, ***caractérisé* en ce que** la tige de blocage (15) est assujettie en partie supérieure à une gâchette (16) pour le passage de la tige de blocage (15) de la position neutre jusqu'à la position de blocage.

6. Ensemble chirurgical (1) selon l'une quelconque des revendications 1 à 5, ***caractérisé* en ce que** la tige de blocage comprend un embout de vissage apte à venir en engagement, en position de blocage, dans l'évidement central (4) pour le vissage du bouchon (2).

7. Ensemble chirurgical (1) selon l'une quelconque des revendications 3 à 6 ***caractérisé* en ce que** les pattes de serrage (13) sont plus longues que les ergots (12) afin de permettre le guidage de l'engagement desdits ergots (12) dans les encoches (7).

8. Ensemble chirurgical (1) selon l'une quelconque des revendications 1 à 7, ***caractérisé* en ce que** le bouchon (2) est constitué de deux bouchons vissés l'un dans l'autre, un bouchon externe (6) comprenant les encoches (7), et un bouchon interne (5) comprenant l'évidement central (4) de vissage.

## Patentansprüche

1. Chirurgischer Bausatz (1), umfassend:
- eine Pedikelschraubenkappe (2), wobei die Kappe (2) mindestens ein externes Gewinde (2c) und eine zentrale Aussparung (4) zum Einsetzen eines Schraubbits umfasst;
- einen Kappenhalter (3) in Form eines hohlen Griffs (10), umfassend ein Verbindungsende (11) mit der Kappe (2) und einen Sperrstift (15) im hohlen Griff (10) verschiebbar zwischen einer Nullstellung und einer Sperrstellung der Kappe (2); und
. die Kappe (2) umfasst mindestens zwei radiale Kerben (7), diametral entgegengesetzt, ausgespart aus einer ersteren Seitenwand dieser Kappe (2), jede der Kerben (7) umfasst zwei entgegengesetzte seitliche Rillen (8);
. das Verbindungsende (11) des Griffs (10) des Kappenhalters (3) verlängert sich in Längsrichtung durch zwei diametral gegenüberliegende Nasen (12), die dafür vorgesehen sind in die Kerben (7) einzurasten und jeweils zwei gegenüberliegende Schultern (12a) umfassen, die zu den Rillen (8) der Kerben (7) komplementär sind und in den Rillen (8) einrasten können, um den Griff (10) an der Kappe (2) zu blockieren;
***dadurch gekennzeichnet, dass***
das Verbindungsende (11) des Griffs (10) zwei längslaufende Schlitze (14) umfasst, die diametral einander gegenüberliegen, so dass sie zwei elastische Äste (11a) bilden, wobei diese Schlitze (14) durch die Nasen (12) führen und damit zwei Paar Nasen (12) bilden, jede Nase (12) jedes Nasenpaares (12) kann, indem sie sich einander durch elastische Verformung der Äste (11a) annähern, in die entsprechende Kerbe (7) einrasten, bis die Schultern (12a) der Nasen (12) in die Rillen (8) der Kerben (7) einklicken.

2. Chirurgischer Bausatz (1) gemäß Anspruch 1, ***dadurch gekennzeichnet, dass*** die Rillen (8) der Kerben (7) und die Schultern (12a) der Nasen (12) in einer Art und Weise geneigt sind, dass die Rotation des Griffs (10) in Schraubrichtung eine axiale Blockade erzwingt.

3. Chirurgischer Bausatz (1) gemäß Anspruch 1, ***dadurch gekennzeichnet, dass*** die Kappe (2) zwei zusätzliche radiale Kerben (7) umfasst, die einander diametral gegenüberliegen und gleichzeitig beiderseits der ersten Kerben (7) verteilt sind und der Griff (10) Verankerungsklauen (13) umfasst, die in Längsrichtung das Verbindungsende (11) des Griffs (10) verlängern, diametral einander gegenüberliegend und gleichmäßig beiderseits der Nasen (12) verteilt, diese Verankerungsklauen (13) sind dazu vorgesehen, passend in die Kerben (7) einzurasten.

4. Chirurgischer Bausatz (1) nach einem der vorstehenden Ansprüche 1 bis 3, ***dadurch gekennzeichnet, dass*** der Sperrstift (15) einen Außendurchmesser aufweist, der an den Innendurchmesser des hohlen Griffs (10) angepasst ist, so dass in der Sperrposition der Sperrstift zwischen den elastischen Ästen (11a) des Griffs (10) liegt, und das Lösen des Kappenhalters (3) verhindert, indem eine Annäherung der elastischen Äste (11a) und eine Entfernung der Nasenpaare (12) verhindert wird.

5. Chirurgischer Bausatz (1) nach einem der Ansprüche 1 bis 4, ***dadurch gekennzeichnet, dass*** der Sperrstift (15) im oberen Teil von einem Drücker (16) abhängig ist, damit der Sperrstift (15) aus der Nullstellung in die Sperrposition gelangen kann.

6. Chirurgischer Bausatz (1) nach einem der Ansprüche 1 bis 5, ***dadurch gekennzeichnet, dass*** der Sperrstift einen Schraubbit umfasst, der in der Sperrposition in die zentrale Aussparung (4) einrasten kann, zum Einschrauben der Kappe (2).

7. Chirurgischer Bausatz (1) nach einem der Ansprüche 3 bis 6, ***dadurch gekennzeichnet, dass*** die Verankerungsklauen (13) länger als die Nasen (12) sind, um eine Führung dieser Nasen (12) in den Kerben (7) zu ermöglichen.

8. Chirurgischer Bausatz (1) nach einem der Ansprüche 1 bis 7, ***dadurch gekennzeichnet, dass*** die Kappe (2) aus zwei ineinander geschraubten Kappen besteht, einer Außenkappe (6), die die Kerben (7) enthält und einer Innenkappe (5), die die zentrale Verschraubungsvertiefung (4) enthält.

## Claims

1. A surgical assembly (1), comprising:
- a pedicle-screw cap (2), where the cap (2) comprises at least one external thread (2c) and a central recess (4) in order to insert a screw bit;
- a cap-holder (3) in the form of a hollow sleeve (10) comprising an end for connection (11) with the cap (2) and a locking pin (15) mounted slidably in the hollow sleeve (10) between a neutral position and a position for locking of the cap (2); and
• the cap (2) includes at least two diametrically opposed radial notches (7), provided from one external lateral wall of said cap (2), each of the notches (7) comprising two opposed lateral grooves (8);
• the connection end (11) of the cap-holder (3) sleeve (10) is extended longitudinally by two diametrically opposed lugs (12) which can engage in the notches (7) and each comprising two opposed lateral shoulders (12a) complementarity to the grooves (8) of the notch (7) and able to engage in said grooves (8) for axially locking the sleeve (10) on the cap (2);
***characterized* in that** the connection end (11) of the sleeve (10) comprises two longitudinal and diametrically opposed slits (14) so as to form two elastic branches (11a), where said slits (14) pass by the lugs (12) forming two pairs of lugs (12), where the lugs (12) from each pair of lugs (12) are able to come to engage, by coming closer to each other by elastic deformation of the branches (11a), in the corresponding notch (7) until the shoulders (12a) of the lugs (12) come to latch in the grooves (8) of the notches (7).

2. The surgical assembly (1) according to claim 1, ***characterized* in that** the grooves (8) of the notches (7) and the shoulders (12a) of the lugs (12) are inclined such that rotation of the sleeve (10) in the direction of screwing forces the axial locking.

3. The surgical assembly (1) according to claim 1, ***characterized* in that** the cap (2) comprises two additional radial notches (7), diametrically opposed and regularly distributed on either side of the first notches (7), and the sleeve (10) comprises tightening tabs (13) which longitudinally extend the connection end (11) of the sleeve (10), and which are diametrically opposed and regularly distributed on either side of the lugs (12), where said tightening tabs (13) are intended to come snugly engage in the notches (7).

4. The surgical assembly (1) according to any one of claims 1 to 3, ***characterized* in that** the locking pin (15) comprises an outer diameter fitted to the inner diameter of the hollow sleeve (10), such that in locking position the locking pin is positioned between the elastic branches (11a) of the sleeve (10) and prevents the unlocking of the cap-holder (3) by preventing the elastic branches (11a) from getting closer and the pairs of lugs (12) from disengaging.

5. The surgical assembly (1) according to any one of claims 1 to 4, ***characterized* in that** the locking pin (15) is subject in the upper part to a latch (16) for the passage of the locking pin (15) from the neutral position to the locking position.

6. The surgical assembly (1) according to any one of claims 1 to 5, ***characterized* in that** the locking pin comprises a screw bit able to come engage, in locking position, in the central recess (4) for screwing the cap (2).

7. The surgical assembly (1) according to any one of claims 3 to 6, ***characterized* in that** the tightening tabs (13) are longer than the lugs (12) in order to serve to guide the insertion of the lugs (12) into the notches (7).

8. The surgical assembly (1) according to any one of claims 1 to 7, ***characterized* in that** the cap (2) is made up of two caps screwed into each other, where an outer cap (6) comprises the notches (7), and an inner cap (5) comprises the central recess (4) for screwing.
